# EUROPEAN PATENT APPLICATION

(11) **EP 4 357 785 A1**
(43) Date of publication of application: **24.04.2024**
(21) Application number: 22202944.9
(22) Date of filing: 21.10.2022
(51) Int. Cl.: G01N 35/00

(54) **LABORATORY INSTRUMENT**

(71) Applicant: F. Hoffmann-La Roche AG, 4070 Basel (CH); Roche Diagnostics GmbH, 68305 Mannheim (DE)
(72) Inventor: Birkner, Patrick, 6343 Rotkreuz (CH)
(74) Representative: Altmann Stößel Dick Patentanwälte PartG mbB

(57) **Abstract**

A laboratory instrument (110) for processing a sample is proposed. The laboratory instrument (110) comprises at least one level sensor (112) configured for generating at least one level signal. The laboratory instrument (110) further comprises at least one evaluation and control unit (114) configured for evaluating the level signal thereby determining at least one item of tilt information on the laboratory instrument (110). The evaluation and control unit (114) is further configured for determining at least one change in the level signal thereby detecting interfering influences on the laboratory instrument (110).

## Description

### Technical Field

The present invention relates to the field of automated in vitro diagnostic (IVD) sample processing. Specifically, the present invention relates to a laboratory instrument for processing a sample and a method for detecting at least one interfering influence on the laboratory instrument. The present invention further relates to a computer program and a computer-readable storage medium for performing the method. The laboratory instrument may, as an example, be used for analyzing samples of a human body, such as blood, urine, saliva, interstitial fluid or other body fluids, tissue section, reagents, system fluids.

### Background art

In diagnostic laboratories, automated pre-analytical, analytical, and post-analytical laboratory instruments are used for various sample processing steps to produce accurate and reliable test results which represent pivotal information for physicians. Such laboratory instruments usually comprise pipetting stations configured for performing pipetting steps such as aspiration, dispensing and the like on one or more of sample, reagent and controls. The sample, reagent and controls are generally handled in single tubes or multiwall plates, sample racks and/or reagent racks which are accommodated in dedicated units assembled on a working surface within the laboratory instrument. A leveled working surface of the laboratory instrument may be crucial in order to ensure high accuracy and reliability of the performance of the laboratory instruments, specifically of the pipetting stations.

GB2571254A discloses a method and an apparatus for monitoring operation of a diagnostic device to determine the health of the device. The method comprises recording, using one or more vibrational sensors, vibrational movements produced during the operation of the diagnostic device and comparing the recorded vibrational movements with stored vibrational patterns in a database. One or more status messages are generated on a display device based on the comparison.

US 2014/0069195 A1 discloses an analyzer, such as a circuit breaker analyzer, a method, and a system for use in determining the mechanical condition of a device, such as a circuit breaker, appliance, machine, equipment, or other mechanical system. In one embodiment the analyzer is implemented using a smartphone or other smart device to couple to the device being analyzed to measure mechanical vibrations generated at a surface of the device during an operational event using a force detector, such as an accelerometer, and then comparing such measured values to a known, good signature of mechanical vibrations for the same type of operational event for same type of equipment.

Despite the advantages achieved by known methods and devices, several technical challenges remains. Specifically, during operation of the laboratory instrument, mechanical shocks or strong vibrations due to improper handling of the laboratory instrument may lead to false analysis results, such due to cross contamination as sample material might splash or spill. Additionally, pipetting errors may occur due loss of position control of pipetting relevant motors. Further, during transport, the laboratory instrument may be damaged by strong mechanical shocks.

### Problem to be solved

It is therefore desirable to provide methods and devices which at least partially address above-mentioned technical challenges. Specifically, a laboratory instrument and a method for detecting at least one interfering influence on the laboratory instrument shall be disclosed which ensure accurate and reliable performance of the laboratory instrument.

### Summary

This problem is addressed by a laboratory instrument, a method for detecting at least one interfering influence on the laboratory instrument, a computer program and a computer-readable storage medium with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, a laboratory instrument for processing a sample is disclosed.

The term "laboratory instrument" as used herein is a broad term and is to be given its ordinary and customary meaning to a person of ordinary skill in the art and is not to be limited to a special or customized meaning. The term specifically may refer, without limitation, to any device configured for performing sample or sample vessel processing steps. For example, the laboratory instrument may be a clinical diagnostic analyzer.

The laboratory instrument may be one or more of a pre-analytical, an analytical, or a post-analytical instrument. A pre-analytical instrument can usually be used for the preliminary processing of samples or sample vessels. An analytical instrument can be designed, for example, to use a sample or part of the sample and a reagent in order to produce a measurable signal, on the basis of which it is possible to determine whether the analyte is present, and if desired in what concentration. A post-analytical instrument can usually be used for the post-processing of samples like the archiving of samples. The laboratory instrument may comprise a pipetting device for pipetting a sample and further devices such as a sorting device for sorting samples or sample vessels, a cap removal device for removing caps or closures on sample vessels, a cap fitting device for fitting caps or closures on sample vessels, an aliquoting device for aliquoting samples, a centrifuging device for centrifuging samples, an analyzing device for analyzing a sample, a heating device for heating a sample, a cooling device for cooling a sample, a mixing device for mixing a sample, a separation device for isolating an analyte of the sample, a storing device for storing samples, an archiving device for archiving samples, a sample vessel type determination device for determining a sample vessel type, a sample quality determination device for determining a sample quality.

The term "sample" as used herein is a broad term and is to be given its ordinary and customary meaning to a person of ordinary skill in the art and is not to be limited to a special or customized meaning. The term specifically may refer, without limitation, to a material suspected of containing an analyte of interest. The sample can be derived from any biological source, such as a biological material including tissue section, and/or physiological fluid, including, blood, saliva, ocular lens fluid, cerebral spinal fluid, sweat, urine, milk, ascites fluid, mucous, synovial fluid, peritoneal fluid, amniotic fluid, tissue, cells or the like. The sample can be pretreated prior to use, such as preparing plasma from blood, diluting viscous fluids, lysis or the like; methods of treatment can involve filtration, distillation, concentration, inactivation of interfering components, and the addition of reagents. Other samples may be possible such as at least one reagent, or at least one system fluid. A sample may be used directly as obtained from the source or following a pretreatment to modify the character of the sample, e.g. after being diluted with another solution or after having being mixed with reagents e.g. to carry out one or more diagnostic assays like e.g. clinical chemistry assays, immunoassays, coagulation assays, nucleic acid testing, etc.. The term "sample" as used herein is therefore not only used for the original sample but also relates to a sample which has already been processed such as pipetted, diluted, mixed with reagents, enriched, having been purified, having been amplified and the like. As used herein, the term "analyte" may refer to the compound or composition to be detected or measured.

The term "processing a sample" as used herein is a broad term and is to be given its ordinary and customary meaning to a person of ordinary skill in the art and is not to be limited to a special or customized meaning. The term specifically may refer, without limitation, to any treatment of the sample. For example, the processing may comprise one or more of transferring, aliquoting, isolating, purifying, incubating, reacting a sample or combining a reagent with a sample. The term "reagent" as used herein is a broad term and is to be given its ordinary and customary meaning to a person of ordinary skill in the art and is not to be limited to a special or customized meaning. The term specifically may refer, without limitation, to a composition required for treatment of a sample. Reagents may be any liquid, e.g. a solvent or chemical solution, which needs to be mixed with a sample and/or other reagent in order e.g. for a reaction to occur, or to enable detection. A reagent may be for example a diluting liquid, including water, it may comprise an organic solvent, it may comprise a detergent, it may be a buffer. Reagents may also be dry reagents adapted e.g. to be dissolved by a sample, another reagent or a diluting liquid. A reagent in the more strict sense of the term may be a liquid solution containing a reactant, typically a compound or agent capable e.g. of binding to or chemically transforming one or more analytes present in a sample. Examples of reactants are enzymes, enzyme substrates, conjugated dyes, protein-binding molecules, nucleic acid binding molecules, antibodies, chelating agents, promoters, inhibitors, epitopes, antigens, etc.. The term "result of the laboratory instrument" as used herein is a broad term and is to be given its ordinary and customary meaning to a person of ordinary skill in the art and is not to be limited to a special or customized meaning. The term specifically may refer, without limitation, to a qualitative and/or quantitative analytical result.

The laboratory instrument comprises at least one level sensor configured for generating at least one level signal. The laboratory instrument further comprises at least one evaluation and control unit configured for evaluating the level signal thereby determining at least one item of tilt information on the laboratory instrument. The evaluation and control unit is further configured for determining at least one change in the level signal thereby detecting at least one interfering influence on the laboratory instrument.

The term "interfering influence" as used herein is a broad term and is to be given its ordinary and customary meaning to a person of ordinary skill in the art and is not to be limited to a special or customized meaning. The term specifically may refer, without limitation, to any change of condition of at least one element of the laboratory instrument. The interfering influence may be an external influence, e.g. a change of at least one external condition on the laboratory instrument such as by exerting at least one external force on the laboratory instrument. Additionally or alternatively, the interfering influence may be an internal influence such as a change of at least one instrument internal condition such as by exerting at least one force on at least one element of the laboratory instrument caused by another element of the laboratory instrument. The external and/or internal force may be exerted in a short time, e.g. in seconds, millisecond or microseconds. The external and/or internal force may be exerted once or repeatedly. The external and/or internal force may result in a sudden acceleration, also denoted as mechanical shock, of at least one part of the laboratory instrument. During operation of the laboratory instrument an interfering influence can lead to false analysis results e.g. cross contamination as sample material might splash and/or spill, or pipetting errors may occur due loss of position control of pipetting relevant motors. The interfering influences may comprises at least one external influence selected from the group consisting of: a mechanical vibration of the laboratory instrument, specifically an abnormal mechanical vibration; a mechanical shock of the laboratory instrument. Mechanical shocks and/or strong vibrations can occur due to non-proper handling of the laboratory instrument. Mechanical shocks and/or strong vibrations can occur during transport of the laboratory instrument. For example, not-normal mechanical vibrations during instrument operation may be detected. The laboratory instrument may be damaged by strong mechanical shocks. The external influence may be exerted by impact such as when closing drawers, e.g. drawers for solid waste or liquid waste. Other embodiment are feasible. For example, the external influence may be exerted by one or more of vibrations of a surface on which the laboratory instrument is arranged, dropping, shaking, kicking, collisions, and the like. The internal influence may be exerted by at least one first element of the laboratory instrument on at least one second element of the laboratory instrument. For example, the first element may be at least one electromechanical element of the laboratory instrument such as at least one of a motor, a drive system, a centrifuge, a transfer system. The second element may be a working plane, e.g. a working plane adjacent to the electromechanical element which performs a mechanical vibration in response to the internal influence exerted by the electromechanical element. For example, the internal influence may be a mechanical vibration and/or another malfunction resulting in change in operation of the electromechanical element.

The term "level" as used herein is a broad term and is to be given its ordinary and customary meaning to a person of ordinary skill in the art and is not to be limited to a special or customized meaning. The term specifically may refer, without limitation, to a spatial angular position of at least one plane of the laboratory instrument, wherein the plane may be working plane. For example, a deviation from a levelled working plane can lead to that a sample holder or sample rack is oriented obliquely in space. For example, deviations from a levelled working plane can lead to cross contamination as sample material might splash and/or spill.

The level may be a measure for horizontal and/or vertical alignment or deviation from a horizontal and/or vertical alignment of the plane. A deviation from a horizontal alignment may result in a deviation from a vertical alignment, too, and vice versa. The level may be a relative height of at least two points of the plane, wherein height may be the respective distance of a point from a chosen reference surface measured upward along a line perpendicular to that surface. The level may be a tilt of the plane.

The term "level sensor" as used herein is a broad term and is to be given its ordinary and customary meaning to a person of ordinary skill in the art and is not to be limited to a special or customized meaning. The term specifically may refer, without limitation, to a sensor configured for generating at least one level signal. The level sensor may be a sensor for tilt measurement. The level sensor may be a biaxial or triaxial level sensor. The level sensor may comprise one or more of at least one accelerometer. The level sensor may comprise at least one microelectromechanical system (MEMS).

The term "level signal" as used herein is a broad term and is to be given its ordinary and customary meaning to a person of ordinary skill in the art and is not to be limited to a special or customized meaning. The term specifically may refer, without limitation, to a signal indicative of at least one angle of the plane with respect to gravity's direction, e.g. indicative of a horizontal alignment or deviation from a horizontal alignment of the plane. The level signal may be or may comprise at least one electrical signal, such as at least one analogue electrical signal and/or at least one digital electrical signal. For example, the level signal may be or may comprise at least one voltage signal and/or at least one current signal and/or changes in capacity.

As used herein, the term "working plane" is a broad term and is to be given its ordinary and customary meaning to a person of ordinary skill in the art and is not to be limited to a special or customized meaning. The term specifically may refer, without limitation, to a plane mounted within the laboratory instrument which represents the actual surface on which at least one processing step is carried out such as one or more of sample, sample vessel, reagent and/ or reagent vessel processing steps of the laboratory instrument. For this purpose, the working plane may comprise with embedded compartments for accommodating consumables, sample processing devices, at least one pipetting station, waste containers, activation devices, and racks which are required for carrying out the sample, sample vessel, reagent and/ or reagent vessel processing steps. The working plane may comprise one single piece. The working plane can be made of any suitable material with sufficient rigidity (e.g. metal and/ or plastic). The working plane may have different shapes and forms to meet the requirements of the laboratory instrument.

The level sensor may be arranged on a working plane of the laboratory instrument. For example, the level sensor may be arranged at a working plane of the pipetting station. The term "pipetting station" as used herein is a broad term and is to be given its ordinary and customary meaning to a person of ordinary skill in the art and is not to be limited to a special or customized meaning. The term specifically may refer, without limitation, to at least one device or system configured for performing at least one pipetting step comprising at least one transport and/or transfer of liquid or other materials such as dry powder. The pipetting station may be configured for performing at least one pipetting step such as aspiration and/or dispensing. The pipetting station may be an automated pipetting station. Thus, the pipetting procedure may be performed automatically, e.g. without user interaction. However, the pipetting procedure may comprise steps which require manual action such as loading of consumables.

The term "evaluation and control unit" as used herein is a broad term and is to be given its ordinary and customary meaning to a person of ordinary skill in the art and is not to be limited to a special or customized meaning. The term specifically may refer, without limitation, to at least one arbitrary device or system configured for performing the named operations. The evaluation and control unit may comprise at least one data processing device, e.g. at least one processor. The evaluation and control unit may comprise a plurality of layers and/or units e.g. for data processing, for controlling the laboratory instrument, for data management and the like.

The evaluation and control unit may comprise at least one microprocessor and at least one storage unit. The microprocessor may be configured retrieving the level signal from the storage unit and for determining the change in the level signal by analyzing the retrieved level signal. The microprocessor and the level sensor may comprise at least one data interface. The term "data interface" as used herein is a broad term and is to be given its ordinary and customary meaning to a person of ordinary skill in the art and is not to be limited to a special or customized meaning. The term specifically may refer, without limitation, to an item or element forming a boundary configured for transferring information. The data interface may be configured for transferring information from the level sensor onto the evaluation and control unit. The data interface may provide means for transferring or exchanging information. The data interface may be designed depending on the level sensor, e.g. in case of an analog or digital level sensor. For example, the data interface may be in case of an analog level sensor an interface for one or more of voltage, current, frequency, capacity and the like, or in case of a digital level sensor one or more of at least one inter-integrated circuit (I2C), Serial Peripheral Interface (SPI), or the like. The storage unit may comprise an electrically erasable programmable read-only memory (EEPROM). The storage unit may be configured for receiving the level signal from the level sensor and for storing the level signal.

The term "item of tilt information" as used herein is a broad term and is to be given its ordinary and customary meaning to a person of ordinary skill in the art and is not to be limited to a special or customized meaning. The term specifically may refer, without limitation, to information about a spatial angular position of at least one plane of the laboratory instrument. The item of tilt information may comprise at least one item of information selected from the group consisting of: a tilt of the laboratory instrument, specifically a tilt of a working plane of the laboratory instrument; a horizontal level of the laboratory instrument.

The term "evaluating the level signal" as used herein is a broad term and is to be given its ordinary and customary meaning to a person of ordinary skill in the art and is not to be limited to a special or customized meaning. The term specifically may refer, without limitation, to applying methods for deriving at least one measurement result from the level signal. The evaluating may comprise pre-processing the level signal such as by filtering, background reduction, signal adjustment, offset adjustment, and the like. The evaluation may comprise processing the level signal e.g. by using at least one algorithm for determining the item of tilt information.

The evaluation and control unit is further configured for determining at least one change in the level signal thereby detecting at least one interfering influence on the laboratory instrument. The mechanical shock and/or vibrations of the laboratory instrument may be detected by using the same sensor as the level sensor, e.g. at least one MEMS. Thus, the detection of at least one interfering influence may be performed using the level sensor. The evaluation and control unit may be configured for comparing the level signal and/or the information derived therefrom to at least one threshold. For example, the threshold may be an allowable deviation, e.g. more than 10 % from an expected value. A change in signal level may be a deviation which exceed the threshold. Additionally or alternatively, the determining of the change in level signal may comprise comparing the level signal at at least two different time points. A change in signal level may be a deviation between at least one first level signal and at least one second level signal which exceed at least one tolerance range. One of the first or the second level signal may be a reference signal.

The evaluation and control unit may comprise at least one instrument control unit for executing at least one instrument control software. The data is stored in the instrument control software (IC-Level). The detected interfering influence on the laboratory instrument and results of the laboratory instrument may be stored in the instrument control software. The evaluation and control unit may be configured for executing at least one action in case at least one interfering influence is detected. The results generated during the time of this shock incidence may be flagged. The evaluation and control unit may be configured for flagging results of the laboratory instrument generated during detected the interfering influence.

The evaluation and control unit further may comprise at least one instrument management unit for executing at least one instrument management software. The instrument management software may be configured for assessing the detected interfering influence on the laboratory instrument stored in the instrument control software and to determine whether results of the laboratory instrument were affected by the detected interfering influence. The instrument management software may be configured for checking, whether a specific result of the laboratory instrument have been affected by the interfering influence, e.g. by shaking the laboratory instrument during a pipetting process.

The laboratory instrument may comprise at least one user interface for displaying the item of tilt information to a user of the laboratory instrument. The term "user interface" as used herein is a broad term and is to be given its ordinary and customary meaning to a person of ordinary skill in the art and is not to be limited to a special or customized meaning. The term may refer, without limitation, to a feature of the laboratory instrument which is configured for interacting with its environment, such as for the purpose of unidirectionally or bidirectionally exchanging information, such as for exchange of one or more of data or commands. For example, the user interface may be configured to share information with a user and to receive information by the user. The user interface may be a feature to interact visually with a user, such as a display, or a feature to interact acoustically with the user. The user interface, as an example, may comprise one or more of a graphical user interface; a data interface, such as a wireless and/or a wire-bound data interface.

For example, the user interface comprises at least one photoelectrical display. The photoelectrical display may comprise a plurality of light emitting diodes (LED), e.g. four, eight or even more LEDs, e.g. for indicating a tilt of a working plane of the laboratory instrument. Each LED may be associated with a degree of freedom for adjusting and/or levelling the working plane.. For example, the laboratory instrument may comprise a plurality of adjustable instrument feet, e.g. one instrument foot at each corner. Each of the LEDs may be associated with an instrument foot. Each LED may be configured for indicating the item of tilt information for the associated sector of the laboratory instrument. Each LED may be configured for emitting light in a color depending on the item of tilt information. For example, each LED may be configured for changing color, e.g. turning from red to green or vice versa, if the working plane of the laboratory instrument is leveled out horizontally. For example, the photoelectrical display may comprise eight LEDs, e.g. four red and four green LEDs. The level sensor may be configured for feeding the level signals to the instrument control software and to the photoelectrical display.

For example, the laboratory instrument may comprise a plurality of level sensors. The level sensors may be arranged on different planes, e.g. on different working planes or on different modules, of the laboratory instrument. The user interface may comprise the at least one photoelectrical display. The photoelectrical display may comprise a plurality of light emitting diodes (LED) e.g. for indicating a tilt of the planes. Each LED may be associated with a plane of the laboratory instrument. Each LED may be configured for indicating the item of tilt information for the associated plane of the laboratory instrument. Each LED may be configured for emitting light in a color depending on the item of tilt information. For example, each LED may be configured for changing color, e.g. turning from red to green or vice versa, if the associated plane of the laboratory instrument is leveled out horizontally. The level sensor may be configured for feeding the level signals to the instrument control software and to the photoelectrical display.

The photoelectrical display may be located at the bottom of a front side of the laboratory instrument. The front side of the laboratory instrument may be the side of the instrument, at which the user can access the laboratory instrument. The back may be a side of the laboratory instrument opposing the front side. The LEDs may change color, when the plane is leveled out horizontally.

The laboratory instrument may comprise the plurality of instrument feet, e.g. four instrument feet. The instrument feet may be adjustable by using a spur wheel mechanism. A height of the instrument feet, e.g. two in a back and two in a front of the laboratory instrument, can be adjusted, e.g. by turning screws of the respective instrument feet. The screws for height adjustment of the instrument feet may be located at the bottom of the front side of the laboratory instrument. A field service engineer can level out the working plane using the color change of the LEDs. A best level of the plane can be preset during assembly in production. This may allow reaching the same level again after adjusting the instruments level at the customer site.

The leveling may be done by the field service technician which will be supported by an LED visualization to ensure proper adjustment as defined during initial production.

The user interface may further be configured for indicating that an interfering influence is detected. For example, the photoelectrical display may further be configured for indicating that an interfering influence is detected e.g. by using a color change, blinking light or the like. The user interface may comprise at least one display. The indication that an interfering influence is detected may be provided to the user by using the display, e.g. by indicating the flagging of the result of the laboratory instrument, and/or by issuing a message such as a warning. For example, the message may comprise an indication to conduct preventive maintenance.

Using a microcontroller, an EEPROM and a MEMS sensor for detection of at least one interfering influence allows for providing a solution in a suitable size and price-range and visualization at a different location than at the location of the sensor.

In a further aspect a method detecting at least one interfering influence on a laboratory instrument is disclosed. The laboratory instrument is configured for processing a sample. The laboratory instrument comprises at least one level sensor and at least one evaluation and control unit. The method comprises using a laboratory instrument according to the present invention, such as according to one or more of the embodiments given above or given in further detail below. For details, options and definitions, reference may be made to the laboratory instrument as discussed above.

The method steps may be performed in the given order or may be performed in a different order. Further, one or more additional method steps may be present which are not listed. Further, one, more than one or even all of the method steps may be performed repeatedly.

The method comprises the following steps:
i) generating, by using the level sensor, at least one level signal;
ii) determining, by using the evaluation and control unit, at least one item of tilt information on the laboratory instrument by evaluating the level signal; and
iii) detecting, by using the evaluation and control unit, at least one interfering influence on the laboratory instrument by determining at least one change in the level signal.

The method may be at least partially computer-implemented. For example, at least one of steps ii) and iii) is computer-implemented. The method may be performed automatically. The term "automatically" as used herein is a broad term and is to be given its ordinary and customary meaning to a person of ordinary skill in the art and is not to be limited to a special or customized meaning. The term specifically may refer, without limitation, to a process which is performed by means of at least one computer and/or computer network and/or machine, for example without manual action and/or interaction with a user. The method may, however, comprise manual action such as loading of consumables and the like.

For example, steps i) to iii) may be performed event-related. For example, the method may be performed for initial setup of the laboratory instrument. For example, the method may be performed during operation of the laboratory instrument. Step i) may comprise continuously and/or repeatedly generating the level signal by using the level sensor. For example, a measurement frequency may be of about 50 Hz. Steps ii) and/or iii) may be performed at least once within a given time interval. For example, steps ii) and/or iii) may be performed at least once within a time interval of one level signal. Steps ii) and iii) may be performed at least partially in a timely overlapping fashion.

Further disclosed and proposed herein is a computer program including computer-executable instructions for performing the method according to the present invention in one or more of the embodiments enclosed herein when the instructions are executed on a computer or computer network. Specifically, the computer program may be stored on a computer-readable data carrier and/or on a computer-readable storage medium.

The computer program comprises instructions which, when the program is executed by the laboratory instrument according to present invention, cause the laboratory instrument, e.g. the evaluation and control unit, to perform at least steps ii) and iii) of the method for detecting at least one interfering influence on a laboratory instrument according to the present invention. The computer program may further comprising instructions which, when the program is executed by the laboratory instrument according to the present invention, e.g. by the evaluation and control unit, cause the laboratory instrument, e.g. the level sensor, to perform step i) of the method for detecting at least one interfering influence on a laboratory instrument according to the present invention.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps i) to iii) as indicated above may be performed by using a computer or a computer network, preferably by using a computer program.

Further disclosed and proposed herein is a computer program product having program code means, in order to perform the method according to the present invention in one or more of the embodiments enclosed herein when the program is executed on a computer or computer network. Specifically, the program code means may be stored on a computer-readable data carrier and/or on a computer-readable storage medium.

The computer-readable storage medium, specifically a non-transient computer-readable storage medium, may comprise instructions which, when the instructions are executed by the laboratory instrument according to the present invention, cause the laboratory instrument to perform at least steps ii) and iii) of the method for detecting at least one interfering influence on a laboratory instrument according to the present invention. The computer-readable storage medium, specifically a non-transient computer-readable storage medium, may comprise instructions which when the instructions are executed by the laboratory instrument according to the present invention, cause the laboratory instrument, e.g. the level sensor, to perform step i) of the method for detecting at least one interfering influence on a laboratory instrument according to the present invention.

Further disclosed and proposed herein is a data carrier having a data structure stored thereon, which, after loading into a computer or computer network, such as into a working memory or main memory of the computer or computer network, may execute the method according to one or more of the embodiments disclosed herein.

Further disclosed and proposed herein is a non-transient computer-readable medium including instructions that, when executed by one or more processors, cause the one or more processors to perform the method according to the present invention.

Further disclosed and proposed herein is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Finally, disclosed and proposed herein is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:
- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

Summarizing and without excluding further possible embodiments, the following embodiments may be envisaged:
Embodiment 1. A laboratory instrument for processing a sample, wherein the laboratory instrument comprises at least one level sensor configured for generating at least one level signal, wherein the laboratory instrument further comprises at least one evaluation and control unit configured for evaluating the level signal thereby determining at least one item of tilt information on the laboratory instrument, wherein the evaluation and control unit is further configured for determining at least one change in the level signal thereby detecting at least one interfering influence on the laboratory instrument.
Embodiment 2. The laboratory instrument according to the preceding embodiment, wherein the evaluation and control unit is configured for flagging results of the laboratory instrument generated during detected interfering influence.
Embodiment 3. The laboratory instrument according to any one of the preceding embodiments, wherein the interfering influence comprises at least one external influence selected from the group consisting of: a mechanical vibration of the laboratory instrument, specifically an abnormal mechanical vibration; a mechanical shock of the laboratory instrument.
Embodiment 4. The laboratory instrument according to any one of the preceding embodiments, wherein the evaluation and control unit comprises at least one microprocessor and at least one storage unit.
Embodiment 5. The laboratory instrument according to the preceding embodiment, wherein the storage unit comprises an electrically erasable programmable read-only memory (EEPROM).
Embodiment 6. The laboratory instrument according to any one of the two preceding embodiments, wherein the storage unit is configured for receiving the level signal from the level sensor and for storing the level signal.
Embodiment 7. The laboratory instrument according to the preceding embodiment, wherein the microprocessor is configured retrieving the level signal from the storage unit and for determining the change in the level signal by analyzing the retrieved level signal.
Embodiment 8. The laboratory instrument according to any one of the preceding embodiments, wherein the evaluation and control unit comprises at least one instrument control unit for executing at least one instrument control software, wherein the detected interfering influence on the laboratory instrument and results of the laboratory instrument are stored in the instrument control software.
Embodiment 9. The laboratory instrument according to the preceding embodiment, wherein the evaluation and control unit further comprises at least one instrument management unit for executing at least one instrument management software, wherein the instrument management software is configured for assessing the detected interfering influence on the laboratory instrument stored in the instrument control software and to determine whether results of the laboratory instrument were affected by the detected interfering influence.
Embodiment 10. The laboratory instrument according to any one of the preceding embodiments, wherein the level sensor comprises at least one microelectromechanical system (MEMS).
Embodiment 11. The laboratory instrument according to any one of the preceding embodiments, wherein the level sensor is arranged on a working plane of the laboratory instrument.
Embodiment 12. The laboratory instrument according to any one of the preceding embodiments, wherein the item of tilt information comprises at least one item of information selected from the group consisting of: a tilt of the laboratory instrument, specifically a tilt of a working plane of the laboratory instrument; a horizontal level of the laboratory instrument.
Embodiment 13. The laboratory instrument according to any one of the preceding embodiments, further comprising at least one user interface for displaying the item of tilt information to a user of the laboratory instrument.
Embodiment 14. A method for detecting at least one interfering influence on a laboratory instrument, the laboratory instrument being configured for processing a sample, wherein the laboratory instrument comprises at least one level sensor and at least one evaluation and control unit, wherein the method comprises the following steps:
   i) generating, by using the level sensor, at least one level signal;
   ii) determining, by using the evaluation and control unit, at least one item of tilt information on the laboratory instrument by evaluating the level signal; and
   iii) detecting, by using the evaluation and control unit, at least one interfering influence on the laboratory instrument by determining at least one change in the level signal.
Embodiment 15. The method according to the preceding embodiment, wherein the method comprises using a laboratory instrument according to any one of the preceding embodiments referring to a laboratory instrument.
Embodiment 16. The method according to anyone of the preceding method embodiments, wherein the method is at least partially computer-implemented, e.g. at least one of steps ii) and iii).
Embodiment 17. A computer program comprising instructions which, when the program is executed by the laboratory instrument according to any one of the preceding embodiments referring to a laboratory instrument, cause the laboratory instrument to perform the method for detecting at least one interfering influence on a laboratory instrument according to any one of the preceding embodiments referring to a method.
Embodiment 18. A computer-readable storage medium, specifically a non-transient computer-readable storage medium, comprising instructions which, when the instructions are executed by the laboratory instrument according to any one of the preceding embodiments referring to a laboratory instrument, cause the laboratory instrument to perform the method for detecting at least one interfering influence on a laboratory instrument according to any one of the preceding embodiments referring to a method.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an embodiment of a laboratory instrument;
- Figure 2: a view to the front side of the laboratory instrument; and
- Figure 3: shows a flow diagram of an embodiment of a method for detecting at least one interfering influence on a laboratory instrument.

### Detailed description of the embodiments

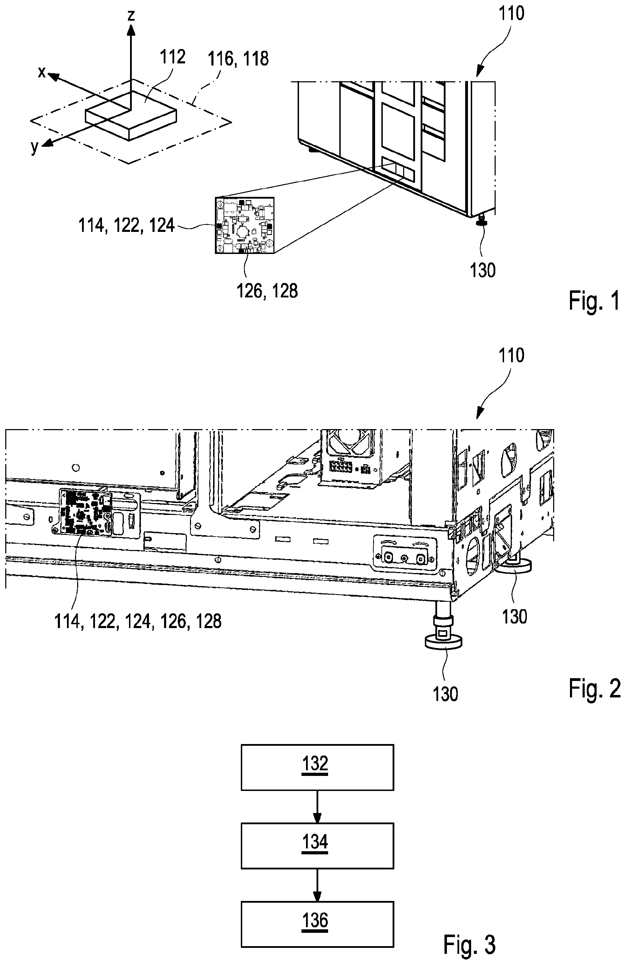

Figure 1 shows a section of an embodiment of a laboratory instrument 110 for processing a sample in a perspective view. A lower part of a front side of the laboratory instrument 110 and a section of a side of the laboratory instrument 110 is shown.

For example, the laboratory instrument 110 may be a clinical diagnostic analyzer. The laboratory instrument may be one or more of a pre-analytical, an analytical, or a post-analytical instrument. The processing of the sample may comprise one or more of transferring, aliquoting, isolating, purifying, incubating, reacting a sample or combining a reagent with a sample. The sample can be derived from any biological source, such as a biological material including tissue section, and/or physiological fluid, including, blood, saliva, ocular lens fluid, cerebral spinal fluid, sweat, urine, milk, ascites fluid, mucous, synovial fluid, peritoneal fluid, amniotic fluid, tissue, cells or the like. The sample can be pretreated prior to use, such as preparing plasma from blood, diluting viscous fluids, lysis or the like; methods of treatment can involve filtration, distillation, concentration, inactivation of interfering components, and the addition of reagents. Other samples may be possible such as at least one reagent, or at least one system fluid. A sample may be used directly as obtained from the source or following a pretreatment to modify the character of the sample, e.g. after being diluted with another solution or after having being mixed with reagents e.g. to carry out one or more diagnostic assays like e.g. clinical chemistry assays, immunoassays, coagulation assays, nucleic acid testing, etc..

The laboratory instrument 110 comprises at least one level sensor 112 configured for generating at least one level signal. The laboratory instrument 110 further comprises at least one evaluation and control unit 114 configured for evaluating the level signal thereby determining at least one item of tilt information on the laboratory instrument 110. The evaluation and control unit 114 is further configured for determining at least one change in the level signal thereby detecting at least one interfering influence on the laboratory instrument 110.

A level may be a spatial angular position of at least one plane of the laboratory instrument 110. The plane 116 may be working plane 118. An exemplary working plane 116 is shown in Figure 3. In a levelled status, the plane 116 may be horizontal. For example, a deviation from a levelled working plane 118 can lead to that a sample holder or sample rack is oriented obliquely in space. For example, deviations from a levelled working plane 118 can lead to cross contamination as sample material might splash and/or spill. Figure 1 shows an exemplary horizontal plane 116 and directions in x, y, and z on which deviations from a horizontal alignment are possible. The level may be a measure for horizontal and/or vertical alignment or deviation from a horizontal and/or vertical alignment of the plane 116. A deviation from a horizontal alignment may result in a deviation from a vertical alignment, too, and vice versa. The level may be a relative height of at least two points of the plane 116, wherein height may be the respective distance of a point from a chosen reference surface measured upward along a line perpendicular to that surface. The level may be a tilt of the plane 116. The level signal may be a signal indicative of at least one angle of the plane 116 with respect to gravity's direction, e.g. indicative of a horizontal alignment or deviation from a horizontal alignment of the plane 116. The level signal may be or may comprise at least one electrical signal, such as at least one analogue electrical signal and/or at least one digital electrical signal. For example, the level signal may be or may comprise at least one voltage signal and/or at least one current signal and/or changes in capacity. The level sensor 112 may be a sensor for tilt measurement. The level sensor 112 may be a biaxial or triaxial level sensor. In the embodiment shown in Figure 3, the level sensor 112 may comprise at least one microelectromechanical system (MEMS).

The level sensor 112 may be arranged on a working plane 118 of the laboratory instrument. The working plane 118 may be a plane 116 mounted within the laboratory instrument 110 which represents the actual surface on which at least one processing step is carried out such as one or more of sample, sample vessel, reagent and/ or reagent vessel processing steps of the laboratory instrument 110. For this purpose, the working plane 118 may comprise with embedded compartments for accommodating consumables, sample processing devices, at least one pipetting station, waste containers, activation devices, and racks which are required for carrying out the sample, sample vessel, reagent and/ or reagent vessel processing steps. The working plane 118 may comprise one single piece. The working plane 118 can be made of any suitable material with sufficient rigidity (e.g. metal and/ or plastic). The working plane 118 may have different shapes and forms to meet the requirements of the laboratory instrument 110.

For example, as shown in Figure 1, the level sensor 112 may be arranged on a plane 116, e.g. at a working plane 118 of the pipetting station 120. The pipetting station 120 may be at least one device or system configured for performing at least one pipetting step comprising at least one transport and/or transfer of liquid or other materials such as dry powder. The pipetting station 120 may be configured for performing at least one pipetting step such as aspiration and/or dispensing. The pipetting station 120 may be an automated pipetting station. Thus, the pipetting procedure may be performed automatically, e.g. without user interaction. However, the pipetting procedure may comprise steps which require manual action such as loading of consumables.

The evaluation and control unit 114 may comprise at least one data processing device, e.g. at least one processor. The evaluation and control unit 114 may comprise a plurality of layers and/or units e.g. for data processing, for controlling the laboratory instrument 110, for data management and the like. As shown in the embodiment of Figure 1, the evaluation and control unit 114 comprises at least one microprocessor 122 and at least one storage unit 124. The microprocessor 122 may be configured retrieving the level signal from the storage unit 124 and for determining the change in the level signal by analyzing the retrieved level signal. The microprocessor 122 and the level sensor 112 may comprise at least one data interface. The data interface may be configured for transferring information from the level sensor onto the evaluation and control unit. The data interface may provide means for transferring or exchanging information. The data interface may be designed depending on the level sensor 112 , e.g. in case of an analog or digital level sensor. For example, the data interface may be in case of an analog level sensor an interface for one or more of voltage, current, frequency, capacity and the like, or in case of a digital level sensor one or more of at least one inter-integrated circuit (I2C), Serial Peripheral Interface (SPI), or the like. The storage unit 124 may comprise an electrically erasable programmable read-only memory (EEPROM). The storage unit 124 may be configured for receiving the level signal from the level sensor 112 and for storing the level signal.

The item of tilt information may comprise at least one item of information selected from the group consisting of: a tilt of the laboratory instrument 110, specifically a tilt of a working plane 118 of the laboratory instrument 110; a horizontal level of the laboratory instrument 110.

The evaluating the level signal may comprise applying methods for deriving at least one measurement result from the level signal. The evaluating may comprise pre-processing the level signal such as by filtering, background reduction, signal adjustment, offset adjustment, and the like. The evaluation may comprise processing the level signal e.g. by using at least one algorithm for determining the item of tilt information.

The evaluation and control unit 114 is further configured for determining at least one change in the level signal thereby detecting interfering influences on the laboratory instrument 110. The interfering influence may be any change of condition of at least one element of the laboratory instrument 110. The interfering influence may be an external influence, e.g. any change of external condition on the laboratory instrument 110 such as by exerting at least one external force on the laboratory instrument 110. Additionally or alternatively, the interfering influence may be an internal influence such as a change of at least one instrument internal condition such as by exerting at least one force on at least one element of the laboratory instrument 110 caused by another element of the laboratory instrument 110. The external and/or internal force may be exerted in a short time, e.g. in seconds, millisecond or microseconds. The external and/or internal force may be exerted once or repeatedly. The external and/or internal force may result in a sudden acceleration of at least one part of the laboratory instrument 110. During operation of the laboratory instrument 110 interfering influence can lead to false analysis results e.g. cross contamination as sample material might splash and/or spill, or pipetting errors may occur due loss of position control of pipetting relevant motors. The interfering influences may comprises at least one external influence selected from the group consisting of: a mechanical vibration of the laboratory instrument 110, specifically an abnormal mechanical vibration; a mechanical shock of the laboratory instrument 110. Mechanical shocks and/or strong vibrations can occur due to non-proper handling of the instrument. Mechanical shocks and/or strong vibrations can occur during transport of the laboratory instrument 110. For example, not-normal mechanical vibrations during instrument operation may be detected. The laboratory instrument 110 may be damaged by strong mechanical shocks. The external influence may be exerted by impact such as when closing drawers, e.g. drawers for solid waste or liquid waste. Other embodiment are feasible. For example, the external influence may be exerted by one or more of vibrations of a surface on which the laboratory instrument 110 is arranged, dropping, shaking, kicking, collisions, and the like. The internal influence may be exerted by at least one first element of the laboratory instrument 110 on at least one second element of the laboratory instrument 110. For example, the first element may be at least one electromechanical element of the laboratory instrument 110 such as at least one of a motor, a drive system, a centrifuge, a transfer system. The second element may be a working plane, e.g. a working plane 118 adjacent to the electromechanical element which performs a mechanical vibration in response to the internal influence exerted by the electromechanical element. For example, the internal influence may be a mechanical vibration and/or another malfunction resulting in change in operation of the electromechanical element.

The interfering influence such as mechanical shock and/or vibrations of the laboratory instrument 110 may be detected by using the same sensor as the level sensor 112, e.g. at least one MEMS. Thus, the detection of the interfering influence may be performed using the level sensor 112. The evaluation and control unit 114 may be configured for comparing the level signal and/or the information derived therefrom to at least one threshold. For example, the threshold may be an allowable deviation, e.g. more than 10 % from an expected value. A change in signal level may be a deviation which exceed the threshold. Additionally or alternatively, the determining of the change in level signal may comprise comparing the level signal at at least two different time points. A change in signal level may be a deviation between at least one first level signal and at least one second level signal which exceed at least one tolerance range. One of the first or the second level signal may be a reference signal.

The evaluation and control unit 114 may comprise at least one instrument control unit for executing at least one instrument control software. The data is stored in the instrument control software (IC-Level). The detected interfering influence on the laboratory instrument 110 and results of the laboratory instrument 114 may be stored in the instrument control software. The evaluation and control unit 114 may be configured for executing at least one action in case at least one interfering influence is detected. The results generated during the time of this shock incidence may be flagged. The evaluation and control unit 114 may be configured for flagging results of the laboratory instrument 110 generated during detected the interfering influence.

The evaluation and control unit 114 further may comprise at least one instrument management unit for executing at least one instrument management software. The instrument management software may be configured for assessing the detected interfering influence on the laboratory instrument 110 stored in the instrument control software and to determine whether results of the laboratory instrument 110 were affected by the detected interfering influence. The instrument management software may be configured for checking, whether a specific result of the laboratory instrument 110 have been affected by the interfering influence, e.g. by shaking the laboratory instrument 110 during a pipetting process.

The laboratory instrument 110 may comprise at least one user interface 126 for displaying the item of tilt information to a user of the laboratory instrument 110. The user interface 126 is enlarged in Figure 1. The user interface 126 may be a feature to interact visually with a user, such as a display, or a feature to interact acoustically with the user. The user interface 126, as an example, may comprise one or more of a graphical user interface; a data interface, such as a wireless and/or a wire-bound data interface.

For example, the user interface 126 comprises at least one photoelectrical display 128, as shown in Figure 1. The photoelectrical display 128 may comprise a plurality of light emitting diodes (LED), e.g. four, eight or even more LEDs, e.g. for indicating a tilt of a working plane of the laboratory instrument 110. Each LED may be associated with a sector of the laboratory instrument 110. Each LED may be configured for indicating the item of tilt information for the associated sector of the laboratory instrument 110. Each LED may be configured for emitting light in a color depending on the item of tilt information. For example, each LED may be configured for changing color, e.g. turning from red to green or vice versa, if the working plane 118 of the laboratory instrument 110 is leveled out horizontally. For example, the photoelectrical display 128 may comprise eight LEDs, e.g. four red and four green LEDs. The level sensor 112 may be configured for feeding the level signals to the instrument control software and to the photoelectrical display 128. Figure 2 shows a view to a front side of the laboratory instrument. The photoelectrical display 128 may be located at the bottom of the front side of the laboratory instrument 110. The front side of the laboratory instrument 110 may be the side of the instrument, at which the user can access the laboratory instrument 110. The back may be a side of the laboratory instrument 110 opposing the front side. The LEDs may change color, when the plane 116 is leveled out horizontally.

The laboratory instrument 110 may comprise a plurality of instrument feet 130, e.g. four instrument feet. As visible, e.g. in Figures 1 and 2, the instrument feet 130 may be adjustable by using a spur wheel mechanism. A height of the instrument feet 130, e.g. two in a back and two in a front of the laboratory instrument 110, can be adjusted, e.g. by turning screws of the respective instrument feet 130. The screws for height adjustment of the instrument feet may be located at the bottom of the front side of the laboratory instrument 110. A field service engineer can level out the working plane 118 using the color change of the LEDs. A best level of the plane 116 can be preset during assembly in production. This may allow reaching the same level again after adjusting the instruments level at the customer site. The leveling may be done by the field service technician which will be supported by an LED visualization to ensure proper adjustment as defined during initial production.

The user interface 126 may further be configured for indicating that an interfering influence is detected. For example, the photoelectrical display 128 may further be configured for indicating that an interfering influence is detected e.g. by using a color change, blinking light or the like. The user interface 126 may comprise at least one display, not shown here. The indication that an interfering influence is detected may be provided to the user by using the display, e.g. by indicating the flagging of the result of the laboratory instrument 110, and/or by issuing a message such as a warning. For example, the message may comprise an indication to conduct preventive maintenance.

Figure 3 shows a flow diagram of an embodiment of a method for detecting at least one interfering influence on the laboratory instrument 110. The method steps may be performed in the given order or may be performed in a different order. Further, one or more additional method steps may be present which are not listed. Further, one, more than one or even all of the method steps may be performed repeatedly.

The method comprises the following steps:
i) (132) generating, by using the level sensor 112, at least one level signal;
ii) (134) determining, by using the evaluation and control unit 114, at least one item of tilt information on the laboratory instrument 110 by evaluating the level signal; and
iii) (136) detecting, by using the evaluation and control unit 114, at least one interfering influence on the laboratory instrument 110 by determining at least one change in the level signal.

The method may be at least partially computer-implemented. For example, at least one of steps ii) and iii) is computer-implemented. The method may be performed automatically. The method may be performed by means of at least one computer and/or computer network and/or machine, for example without manual action and/or interaction with a user. The method may, however, comprise manual action such as loading of consumables and the like.

For example, steps i) to iii) may be performed event-related. For example, the method may be performed for initial setup of the laboratory instrument. For example, the method may be performed during operation of the laboratory instrument. Step i) may comprise continuously and/or repeatedly generating the level signal by using the level sensor. For example, a measurement frequency may be of about 50 Hz. Steps ii) and/or iii) may be performed at least once within a given time interval. For example, steps ii) and/or iii) may be performed at least once within a time interval of one level signal. Steps ii) and iii) may be performed at least partially in a timely overlapping fashion.

### List of reference numbers

- 110: laboratory instrument
- 112: level sensor
- 114: evaluation and control unit
- 116: plane
- 118: working plane
- 120: pipetting station
- 122: microprocessor
- 124: storage unit
- 126: user interface
- 128: photoelectrical display
- 130: instrument feet
- 132: Step i)
- 134: Step ii)
- 136: Step iii)

## Claims

1. A laboratory instrument (110) for processing a sample, wherein the laboratory instrument (110) comprises at least one level sensor (112) configured for generating at least one level signal, wherein the laboratory instrument (110) further comprises at least one evaluation and control unit (114) configured for evaluating the level signal thereby determining at least one item of tilt information on the laboratory instrument (110), wherein the evaluation and control unit (114) is further configured for determining at least one change in the level signal thereby detecting at least one interfering influence on the laboratory instrument (110).

2. The laboratory instrument (110) according to the preceding claim, wherein the evaluation and control unit (114) is configured for flagging results of the laboratory instrument (110) generated during detected interfering influence.

3. The laboratory instrument (110) according to any one of the preceding claims, wherein the interfering influence comprises at least one external influence selected from the group consisting of: a mechanical vibration of the laboratory instrument (110); a mechanical shock of the laboratory instrument (110).

4. The laboratory instrument (110) according to any one of the preceding claims, wherein the evaluation and control unit (114) comprises at least one microprocessor (122) and at least one storage unit (124).

5. The laboratory instrument (110) according to the preceding claim, wherein the storage unit (124) comprises an electrically erasable programmable read-only memory (EEPROM).

6. The laboratory instrument (110) according to any one of the two preceding claims, wherein the storage unit (124) is configured for receiving the level signal from the level sensor (112) and for storing the level signal, wherein the microprocessor (122) is configured retrieving the level signal from the storage unit (124) and for determining the change in the level signal by analyzing the retrieved level signal.

7. The laboratory instrument (110) according to any one of the preceding claims, wherein the evaluation and control unit (114) comprises at least one instrument control unit for executing at least one instrument control software, wherein the detected interfering influences on the laboratory instrument (110) and results of the laboratory instrument (110) are stored in the instrument control software, wherein the evaluation and control unit (114) further comprises at least one instrument management unit for executing at least one instrument management software, wherein the instrument management software is configured for assessing the detected interfering influence on the laboratory instrument (110) stored in the instrument control software and to determine whether results of the laboratory instrument (110) were affected by the detected interfering influence.

8. The laboratory instrument (110) according to any one of the preceding claims, wherein the level sensor (112) comprises at least one microelectromechanical system (MEMS).

9. The laboratory instrument (110) according to any one of the preceding claims, wherein the level sensor (112) is arranged on a working plane of the laboratory instrument (110).

10. The laboratory instrument (110) according to any one of the preceding claims, wherein the item of tilt information comprises at least one item of information selected from the group consisting of: a tilt of the laboratory instrument (110), specifically a tilt of a working plane (118) of the laboratory instrument; a horizontal level of the laboratory instrument (110), wherein the laboratory instrument (110) further comprise at least one user interface (126) for displaying the item of tilt information to a user of the laboratory instrument (110).

11. A method for detecting at least one interfering influence on a laboratory instrument (110), the laboratory instrument (110) being configured for processing a sample, wherein the laboratory instrument (110) comprises at least one level sensor (112) and at least one evaluation and control unit (114), wherein the method comprises the following steps:
i) (132) generating, by using the level sensor (112), at least one level signal;
ii) (134) determining, by using the evaluation and control unit (114), at least one item of tilt information on the laboratory instrument (110) by evaluating the level signal; and
iii) (136) detecting, by using the evaluation and control unit (114), at least one interfering influence on the laboratory instrument (110) by determining at least one change in the level signal.

12. The method according to the preceding claim, wherein the method comprises using a laboratory instrument (110) according to any one of the preceding claims referring to a laboratory instrument.

13. The method according to anyone of the preceding method claims, wherein the method is at least partially computer-implemented, e.g. at least one of steps ii) and iii).

14. A computer program comprising instructions which, when the program is executed by the laboratory instrument (110) according to any one of the preceding claims referring to a laboratory instrument, cause the laboratory instrument to perform the method for detecting at least one interfering influence on a laboratory instrument (110) according to any one of the preceding claims referring to a method.

15. A computer-readable storage medium, specifically a non-transient computer-readable storage medium, comprising instructions which, when the instructions are executed by the laboratory instrument (110) according to any one of the preceding claims referring to a laboratory instrument, cause the laboratory instrument (110) to perform the method for detecting at least one interfering influence on a laboratory instrument according to any one of the preceding claims referring to a method.
